# EUROPEAN PATENT APPLICATION

(11) **EP 2 090 314 A1**
(43) Date of publication of application: **19.08.2009**
(21) Application number: 07830401.1
(22) Date of filing: 23.10.2007
(51) Int. Cl.: A61K 31/728, A61K 9/08, A61K 47/12, A61P 27/02

(54) **OPHTHALMIC AQEOUS LIQUID PREPARATION**

(30) Priority: 26.10.2006 JP 2006290826
(71) Applicant: Senju Pharmaceutical Co., Ltd., Osaka-shi, Osaka 541-0046 (JP)
(72) Inventor: YAMAMOTO, Makiko, Kobe-shi Hyogo 6512241 (JP)
(74) Representative: Weber, Thomas
(86) International application number: PCT/JP2007/070667
(87) International publication number: WO 2008/050776

(57) **Abstract**

Disclosed is a method for increasing the stability of viscosity of aqueous eye drops containing hyaluronic acid. The method is **characterized in that** it employs hyaluronic acid or its pharmaceutically acceptable salt and, in addition, gluconic acid or a metal salt thereof, while containing no chlorhexidine or its salt.

## Description

### TECHNICAL FIELD

The present invention relates to an ophthalmic aqueous composition, in particular to an ophthalmic aqueous composition containing hyaluronic acid or a salt thereof.

### BACKGROUND ART

Hyaluronic acid is a linear high molecular-weight polysaccharide consisting of a large number of D-N-acetylglucosamine and D-glucuronic acid molecules which are alternately linked together. Aqueous solution of hyaluronic acid or its salt has been used in aqueous eye drops for such reasons that it is highly viscous, capable of retaining water, and has an activity to promote expansion of the corneal epithelium layer. However, it is known that in an aqueous solution which contains hyaluronic acid or its salt and is buffered around neutral pH, the viscosity given by hyaluronic acid declines during long-time storage. Thus, such a solution has a problem that it cannot maintain its viscosity for an extended period of time.

While it has been disclosed that among cosmetic preparations containing hyaluronic acid or its salt, those which comprise pyrrolidonecarboxylic acid, lactic acid or urea, together with a polyol and an oil, can prevent the viscosity decline with time of hyaluronic acid, no data are provided on the viscosity of those preparations (see Patent document 1).
In addition, although it has been reported that tryptophan, histidine and urea acts to prevent molecular breakdown of hyaluronic acid in those eye drops which contain hyaluronic acid or its salt, no data have been presented regarding the prevention of molecular breakdown (see Patent document 2).
Furthermore, it has been proposed to add a compound having a phenolic hydroxyl group like phenol or cresol, etc. to a solution containing hyaluronic acid or its salt (see Patent document 3). However, such compounds as phenol or cresol irritate the eye, and therefore are not suitable for use in eye drops containing hyaluronic acid.

To address the problem of viscosity decline with time of eye drops containing hyaluronic acid or its salt, it has been proposed that a polyol (glycerol, propylene glycol etc.), a polycarboxylic acid (edetic acid, citric acid, etc.) or their salt or a sugar (sorbitol, etc.) should be added to eye drops which contain a solution of hyaluronic acid or its salt, and among those compounds, addition of glycerol, disodium edetate, sorbitol or propylene glycol has been shown, with experimental data, to hinder viscosity decline with time of a hyaluronic acid-containing eye drops (see Patent document 4).

On the other hand, though aqueous eye drops with good preservative effect are disclosed which contain a cationic surfactant as a preservative (benzalkonium chloride or chlorhexidine gluconate), a buffer, a chelating agent (ethylenediamine tetraacetic acid), and sodium hyaluronate, with their examples 4 and 5 containing benzalkonium chloride being described as stable in their appearance, no reference is made to any stability over time of their viscosity (see Patent documents 5, esp., Table 9).
[Patent document 1] Japanese patent application publication No. S61-180705
[Patent document 2] Japanese patent application publication No. S61-233622
[Patent document 3] Japanese patent application publication No. H1-113401
[Patent document 4] Japanese patent application publication No. H10-72376
[Patent document 5] Japanese patent application publication No. H 10-203960

### DISCLOSURE OF INVENTION

Against the above background, the objective of the present invention is to provide a further method to improve the stability of viscosity of aqueous eye drops containing hyaluronic acid, and thereby to provide novel eye drops containing hyaluronic acid.

### [MEANS TO SOLVE THE PROBLEM]

As a result of studies made toward the above objective the present inventor found that gluconic acid and its salts have an effect of preventing the viscosity decline with time of hyaluronic acid in aqueous solutions, and accomplished the present invention after further studies. Thus, the present invention provides what follows.

(1) An ophthalmic aqueous composition comprising hyaluronic acid or a pharmaceutically acceptable salt thereof and, in addition, gluconic acid or a metal salt thereof, while containing no chlorhexidine or a salt thereof.
(2) The ophthalmic aqueous composition according to (1) above, wherein the metal salt of gluconic acid is at least a compound selected from sodium gluconate, calcium gluconate and magnesium gluconate.
(3) The ophthalmic aqueous composition according to (1) or (2) above comprising gluconic acid or a metal salt thereof in an amount of 0.02-6 parts by weight per one part by weight of hyaluronic acid or a pharmaceutically acceptable salt thereof.
(4) The ophthalmic aqueous composition according to one of (1) to (3) above, wherein the composition is eye drops.
(5) A method for hindering viscosity decline with time of an aqueous solution containing hyaluronic acid or a pharmaceutically acceptable salt thereof, wherein the method comprises adding gluconic acid or a metal salt thereof to the aqueous solution.
(6) The method according to (5) above, wherein the metal salt of gluconic acid is at least a compound selected from sodium gluconate, calcium gluconate and magnesium gluconate.
(7) The method according to (5) or (6) above, wherein gluconic acid or a metal salt thereof is added in an amount of 0.02-6 parts by weight per one part by weight of hyaluronic acid or a pharmaceutically acceptable salt thereof.

### [THE EFFECT OF THE INVENTION]

By hindering viscosity decline with time of hyaluronic acid, the present invention as defined above can provide ophthalmic aqueous compositions containing hyaluronic acid or a salt thereof, whose viscosity is maintained stable even after storage for an extended period of time.

### BEST MODE FOR CARRYING OUT THE INVENTION

Although in the present invention, the concentration of hyaluronic acid or a salt thereof may be adjusted as desired in accordance with the intended viscosity, the concentration, usually, is preferably 0.05-0.4 w/v%, more preferably 0.1-0.3 w/v%. A preferable salt of hyaluronic acid is sodium hyaluronate.

Gluconic acid may be employed either in its free acid form or in the form of its metal salt. Preferred metal salts are alkali metal salts such as sodium salt and potassium salt, and alkaline earth metal salts such as calcium salt and magnesium salt.

As will be seen in the section of "Examples", in terms of the ratio of the amount of gluconic acid or its salt employed to one part by weight of hyaluronic acid or its salt, an effect is observed at the ratio of at least 0.025 in the lower region, and remarkable effect is still observed even at the rate of 2 in the upper region. Thus, gluconic acid or its salt may be employed, per one part by weight of hyaluronic acid or its salt, usually in the amount of 0.02-6 parts by weight preferably 0.1-4 parts by weight, and most preferably 0.2-3 parts by weight.

Though there is no particular limitation regarding the pH of the ophthalmic aqueous composition according to the present invention, its pH may usually be 5-8, preferably 6-8, and most preferably 6.5-7.5, considering that it is an aqueous composition applied to the eye.

The ophthalmic aqueous composition according to the present invention may contain a buffer in order to maintain its pH within a predetermined range, examples which include a borate buffer and a phosphate buffer. Also it may contain, as an isotonizing agent, one of those salts which are conventionally employed in ophthalmic aqueous compositions, such as sodium chloride, potassium chloride, calcium chloride, magnesium chloride, and the like. Other compounds which are generally used in ophthalmic aqueous compositions may also be employed insofar as they do not work contrary to the purpose of the present invention.

### [EXAMPLES]

The present invention will be described in further detail with reference to Comparative examples and Examples. It is not intended, however, that the present invention be limited to the Examples.

### [Example 1]

Ophthalmic aqueous compositions containing sodium hyaluronate (Control composition, Compositions 1-3) in accordance with the formulas presented in Table 1. The control composition was an aqueous composition which contained sodium hyaluronate but no candidate compound for viscosity stabilization, and the compositions 1-3 were compositions prepared by adding to the Control composition α-cyclodextrin, carmellose sodium (CMC), or calcium gluconate, respectively, as a candidate compound for viscosity stabilization. Each of those composition were subjected to an accelerated stability test at 40°C and a stability test under a severe condition at 60°C, and the change of their viscosity was measured using Ubbelohde viscometer (<2.53> Method 1. Capillary viscometer method, General Test Procedures, Japanese Pharmacopoeia,). The results are shown in Tables 2 and 3. In these tables, "viscosity retention rate" means "viscosity after storage/viscosity at the start of the test × 100 (%)".

[TABLE 1]

**Table 1.**

| Ingredients | Control preparation 1 | Preparation 1 | Preparation 2 | Preparation 3 |
|---|---|---|---|---|
| Sodium hyaluronate | 0.1 | 0.1 | 0.1 | 0.1 |
| Sodium chloride | 0.55 | 0.55 | 0.55 | 0.55 |
| Potassium chloride | 0.16 | 0.16 | 0.16 | 0.16 |
| Boric acid | 0.4 | 0.4 | 0.4 | 0.4 |
| Borax | q.s. | q.s. | q.s. | q.s. |
| α-cyclodextrin | - | 0. 05 | - | - |
| Carmellose sodium (CMC)) | - | - | 0. 05 | - |
| Calcium gluconate | - | - | - | 0. 2 |
| Purified water | q.s. | q.s. | q.s. | q.s. |
| pH | 7 | 7 | 7 | 7 |

(For each ingredient, figures are in g/100 mL)

[TABLE 2]

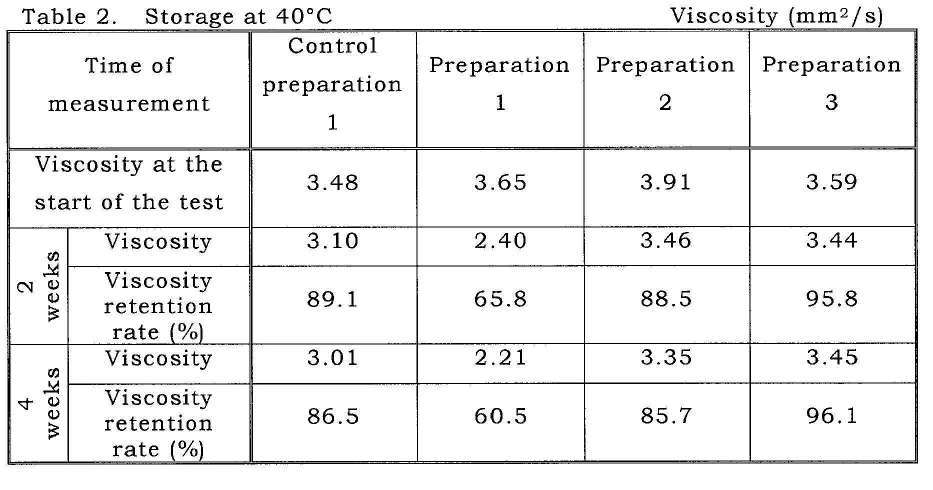

[TABLE 3]

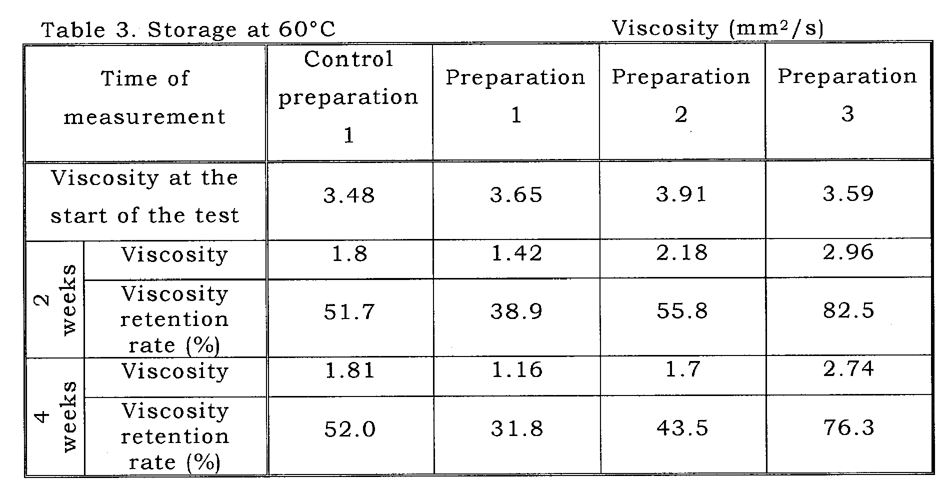

During the storage at 40°C, as shown in Table 2, the viscosity of Control preparation 1 declined to 89.1 % of the initial value in 2 weeks, and to 86.5% of the initial value in 4 weeks. During the storage at 40°C, the viscosity of Preparation 1 (α-cyclodextrin) and Preparation 2 (carmellose sodium) declined to 65.8% and 88.5%, respectively, of their initial values, in 2 weeks, and to 60.5% and 85.7%, respectively, in 4 weeks. This indicates that α-cyclodextrin brings about a greater decline of the viscosity than is observed in Control preparation, acting in the direction to accelerate the decline of viscosity of the hyaluronic acid-containing aqueous solution. With carmellose sodium, the decline of the viscosity was comparable to or less than that was observed in Control preparation 1, thus showing no effect of hindering viscosity decline of the hyaluronic acid-containing aqueous solution. In contrast, Preparation 3 (calcium gluconate) was revealed to maintain much higher viscosity than was observed in Control preparation either after 2 or 4 weeks of storage at 40°C, indicating that calcium gluconate has an effect of hindering viscosity decline of a hyaluronic acid-containing aqueous solution. During the storage at 60°C shown in Table 3, like what was observed in the storage at 40°C, α-cyclodextrin (Preparation 1) acted in the direction to accelerate the viscosity decline of the hyaluronic acid-containing aqueous solution, and carmellose sodium (Preparation 2) did not show any effect of hindering viscosity decline of the hyaluronic acid-containing aqueous solution but even rather accelerated the viscosity decline during the 4-week storage. In contrast, it was observed that 0.2 w/v% calcium gluconate (Preparation 3) had a remarkable effect of hindering viscosity decline of the hyaluronic-acid containing aqueous solution.

### [Example 2]

As calcium gluconate was found to be effective in the above, preparations were provided which contained, in two different ways, each of sodium gluconate, calcium gluconate and magnesium gluconate, and they were tested for their effect of hindering viscosity decline of a hyaluronic acid-containing aqueous solution after subjected to an accelerated stability test and a stability test under a severe condition. Further, Control preparation 3, which contained a different concentration of hyaluronic acid, and Preparation 10, which was the same as Control preparation 3 except that it further contained sodium gluconate, were prepared and subjected to a stability test under a severe condition to examine sodium gluconate for its effect of hindering viscosity decline. The results are shown in Tables 5 and 6.

[TABLE 4]

**Table 4.**

| | | | | | | | | | |
|---|---|---|---|---|---|---|---|---|---|
| (g/100ml) | Control preparation 2 | Preparation 4 | Preparation 5 | Preparation 6 | Preparation 7 | Preparation 8 | Preparation 9 | Control preparation 3 | Preparation 10 |
| Sodium hyaluronate | 0.1 | 0.1 | 0.1 | 0.1 | 0.1 | 0.1 | 0.1 | 0.2 | 0.2 |
| Sodium chloride | 0.55 | 0.55 | 0.55 | 0.55 | 0.55 | 0.55 | 0.55 | 0.55 | 0.55 |
| Potassium chloride | 0.16 | 0.16 | 0.16 | 0.16 | 0.16 | 0.16 | 0.16 | 0.16 | 0.16 |
| Boric acid | 0.4 | 0.4 | 0.4 | 0.4 | 0.4 | 0.4 | 0.4 | 0.4 | 0.4 |
| Borax | q.s. | q.s. | q.s. | q.s. | q.s. | q.s. | q.s. | q.s. | q.s. |
| Calcium gluconate | - | 0.0013 | 0.1 | | - | - | - | - | - |
| Sodium gluconate | - | - | - | 0.0025 | 0.2 | - | - | - | 0.14 |
| Magnesium gluconate | - | - | - | - | - | 0.0013 | 0.1 | - | - |
| Purified water | q.s. | q.s. | q.s. | q.s. | q.s. | q.s. | q.s. | q.s. | q.s. |
| pH | 7 | 7 | 7 | 7 | 7 | 7 | 7 | 7 | 7 |

(For each ingredients, figures are in g/100 mL)

[TABLE 5]

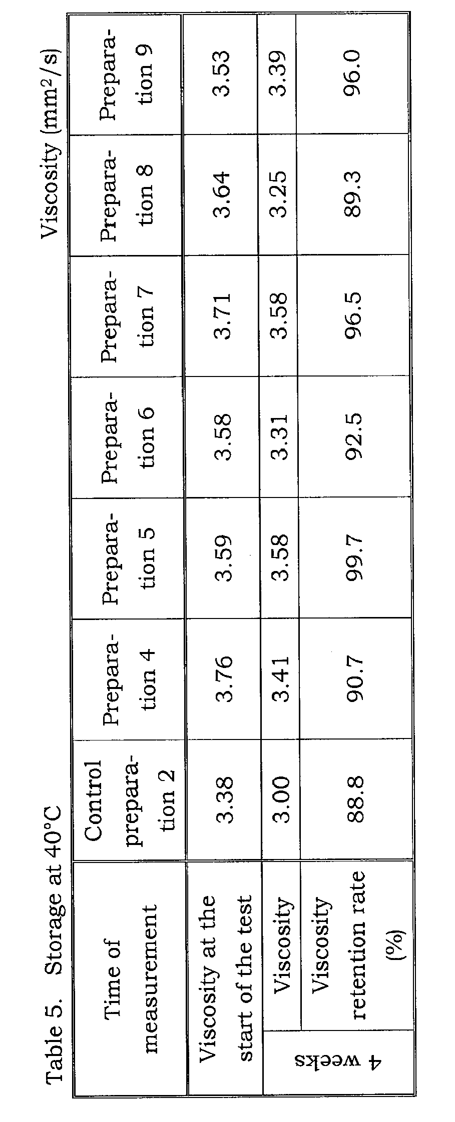

[TABLE 6]

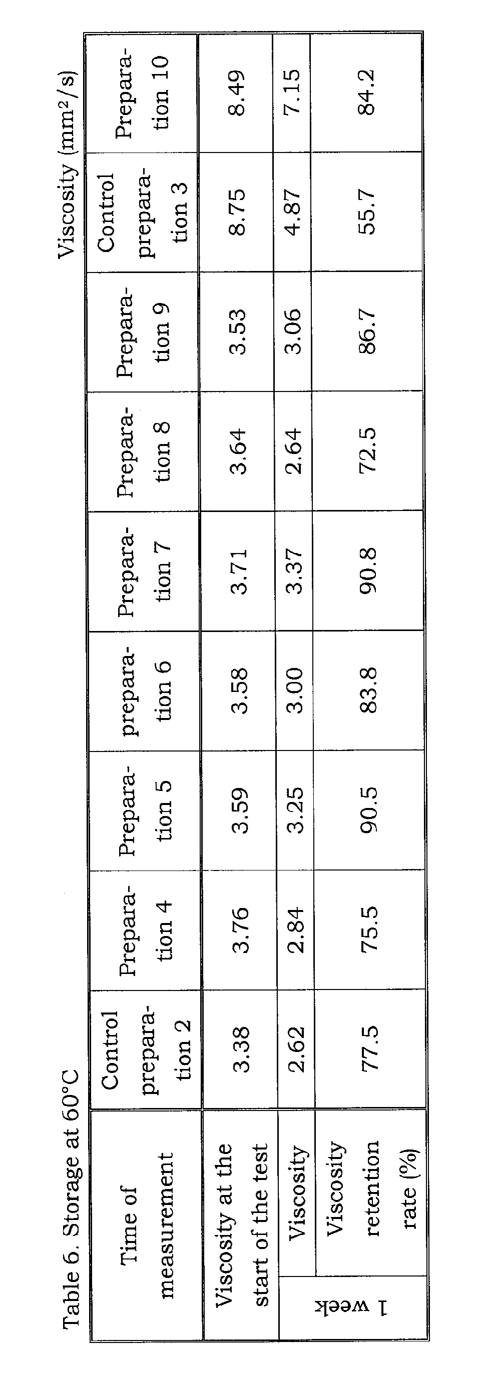

After the 4-week storage at 40°C, as seen in Table 5, Preparations 4 and 8, which contained 0.0013 w/v% calcium gluconate or magnesium gluconate, respectively, were found largely comparable to Control preparation with regard to their viscosity retention rates, thus showing no effect of hindering viscosity decline. In contrast, Preparation 5 (0.1 w/v% calcium gluconate), Preparation 6 (0.0025 w/v% sodium gluconate), Preparation 7 (0.2 w/v% sodium gluconate), and Preparation 9 (0.1% magnesium gluconate) all showed higher viscosity retention rates than Control preparation 2, thereby demonstrating effects of hindering viscosity decline. Thus, it has been shown that at least 0.0025 w/v% of a salt of gluconic acid, or a corresponding amount of gluconic acid, when contained in a aqueous solution which contains 0.1 w/v% hyaluronic acid, is effective in hindering viscosity decline of the solution.

The stability test under a severe condition, i.e., one week storage at 60°C, revealed, as seen in Table 6, that decline of the viscosity was remarkably smaller in Preparation 5, Preparation 6, Preparation 7 and Preparation 9 than in Control preparation 2, as was seen in the result of the accelerated stability test of 4-week storage at 40°C. This result reconfirms the result of the accelerated stability test at 40°C, namely that at least 0.0025 w/v% of a salt of gluconic acid, or a corresponding amount of gluconic acid, when contained per 0.1 w/v% hyaluronic acid, is effective in hindering viscosity decline of the hyaluronic acid-containing aqueous solution.
Further, in the preparations containing 0.2 w/v% sodium hyaluronate (Control preparation 3 and Preparation 10), the viscosity decline after one-week storage at 60°C was confirmed to be remarkably hindered in Preparation 10 (0.14 w/v% sodium gluconate), which contained sodium gluconate in an amount that was 0.7 times that of hyaluronic acid contained in Control preparation 3.

**[Preparation Example 1]**

| | |
|---|---|
| Sodium hyaluronate | 0.1 g |
| Calcium gluconate | 0.05 g |
| Sodium chloride | 0.55 g |
| Potassium chloride | 0.16 g |
| Boric acid | 0.4 g |
| Borax | q.s. |
| Purified water | to 100 mL in total |
| pH | 6 |

According to the above formula, the materials are mixed in a conventional manner to form an aqueous solution, which then is filter-sterilized to give eye drops.

**[Preparation Example 2]**

| | |
|---|---|
| Sodium hyaluronate | 0.1 g |
| Sodium gluconate | 0.5 g |
| Sodium chloride | 0.58 g |
| Potassium chloride | 0.14 g |
| Magnesium chloride | 0.02 g |
| Calcium chloride | 0.006 g |
| Boric acid | 0.229 g |
| Borax | q.s. |
| Purified water | to 100 mL in total |
| pH | 7 |

According to the above formula, the materials are mixed in a conventional manner to form an aqueous solution, which then is filter-sterilized to give eye drops.

**[Preparation Example 3]**

| | |
|---|---|
| Sodium hyaluronate | 0.1 g |
| Magnesium gluconate | 0.2 g |
| Sodium chloride | 0.55 g |
| Potassium chloride | 0.16 g |
| Boric acid | 0.4 g |
| Borax ----- | q.s. |
| Purified water | to 100 mL in total |
| pH | 7 |

According to the above formula, the materials are mixed in a conventional manner to form an aqueous solution, which then is filter-sterilized to give eye drops.

**[Preparation Example 4]**

| | |
|---|---|
| Sodium hyaluronate | 0.2 g |
| Calcium gluconate | 0.3 g |
| Sodium chloride | 0.6 g |
| Boric acid | 0.4 g |
| Borax | q.s. |
| Purified water | to 100 mL in total |
| pH | 7 |

According to the above formula, the materials are mixed in a conventional manner to form an aqueous solution, which then is filter-sterilized to give eye drops.

**[Preparation Example 5]**

| | |
|---|---|
| Sodium hyaluronate | 0.25 g |
| Sodium gluconate | 0.3 g |
| Sodium chloride | 0.6 g |
| Boric acid | 0.4 g |
| Borax | q.s. |
| Purified water | to 100 mL in total |
| pH | 6 |

According to the above formula, the materials are mixed in a conventional manner to form an aqueous solution, which then is filter-sterilized to give eye drops.

**[Preparation Example 6]**

| | |
|---|---|
| Sodium hyaluronate | 0.1 g |
| Sodium gluconate | 0.2 g |
| Sodium chloride | 0.55 g |
| Potassium chloride | 0.16 g |
| Sodium hydrogen phosphate | 0.016 g |
| Sodium dihydrogen phosphate | 0.08 g |
| Borax | q.s. |
| Purified water | to 100 mL in total |
| pH | 7 |

According to the above formula, the materials are mixed in a conventional manner to form an aqueous solution, which then is filter-sterilized to give eye drops.

### INDUSTRIAL APPLICABILITY

The present invention provides a method to increase the stability of the viscosity of hyaluronic acid-containing aqueous eye drops, as well as hyaluronic acid-containing ophthalmic aqueous compositions with hindered viscosity decline which are prepared by the method.

## Claims

1. An ophthalmic aqueous composition comprising hyaluronic acid or a pharmaceutically acceptable salt thereof and, in addition, gluconic acid or a metal salt thereof, while containing no chlorhexidine or a salt thereof.

2. The ophthalmic aqueous composition according to claim 1, wherein the metal salt of gluconic acid is at least a compound selected from sodium gluconate, calcium gluconate and magnesium gluconate.

3. The ophthalmic aqueous composition according to claim 1 or 2 comprising gluconic acid or a metal salt thereof in an amount of 0.02-6 parts by weight per one part by weight of hyaluronic acid or a pharmaceutically acceptable salt thereof.

4. The ophthalmic aqueous composition according to one of claims 1 to 3, wherein the composition is eye drops.

5. A method for hindering viscosity decline with time of an aqueous solution containing hyaluronic acid or a pharmaceutically acceptable salt thereof, wherein the method comprises adding gluconic acid or a metal salt thereof to the aqueous solution.

6. The method according to claim 5, wherein the metal salt of gluconic acid is at least a compound selected from sodium gluconate, calcium gluconate and magnesium gluconate.

7. The method according to claim 5 or 6, wherein gluconic acid or a metal salt thereof is added in an amount of 0.02-6 parts by weight per one part by weight of hyaluronic acid or a pharmaceutically acceptable salt thereof.
